Europäisches Patentamt

European Patent Office

Office européen des brevets

(11) Publication number: **0 257 493**
**A1**

# (12) EUROPEAN PATENT APPLICATION

(21) Application number: 87111837.8

(22) Date of filing: **14.08.87**

(51) Int. Cl.4: **B01D 53/04** , **C01B 3/56** , **C01B 21/04** , **C01B 31/20** , **C07C 9/04**

(30) Priority: **22.08.86 US 899441**

(43) Date of publication of application:
**02.03.88 Bulletin 88/09**

(84) Designated Contracting States:
**BE DE ES FR GB IT NL**

(71) Applicant: **AIR PRODUCTS AND CHEMICALS, INC.**
**P.O. Box 538**
**Allentown, Pennsylvania 18105(US)**

(72) Inventor: **Sircar, Shivaji**
**1508 Bogie Avenue**
**Wescosville,PA 18106(US)**
Inventor: **Koch, William R.**
**Rd. No.1, Box 1479**
**Fleetwood, PA 19522(US)**

(74) Representative: **Kador & Partner**
**Corneliusstrasse 15**
**D-8000 München 5(DE)**

(54) **Adsorptive separation of gas mixtures.**

(57) A process is described for separating gas mixtures containing a primary gaseous component and a secondary gaseous component by selective adsorption of the secondary gaseous component in an adsorptive process including the steps of adsorption in at least one adsorbent bed, rinsing said bed with secondary component, depressurizing and evacuating said bed and repressurizing said bed with primary gaseous component. A key aspect of the process is the utilization of an adsorbent having a selectivity(S) greater than 20 for the gas mixture being resolved at the entire pressure and temperature ranges that the adsorbent in the bed undergoes during separation. The process is particularly attractive for recovering methane (primary component) and carbon dioxide (secondary component) from landfill gas.

*FIG.1*

## ADSORPTIVE SEPARATION OF GAS MIXTURES

### TECHNICAL FIELD

The present application is a continuation-in-part of Application Serial Number 695,151 filed January 25, 1985.

The present invention is directed to the separation of certain gaseous mixtures at high pressure in an adsorptive separation utilizing a pressure swing cycle and a high selectivity adsorbent. More particularly, the present invention is directed to the separation of methane and/or carbon dioxide-containing gas mixtures, such as are recovered in enhanced oil recovery and landfill gas recovery operations.

### BACKGROUND OF THE PRIOR ART

Adsorptive separations using pressure swing cyclic procedures for recovering or isolating components of gas mixtures are well known in the art. These systems typically utilize a series of parallel adsorptive beds operated in a switching pattern wherein at least one bed is in an adsorption sequence, while another bed is in a regeneration sequence. Generally recognized steps in adsorptive separation include adsorption, purging, depressurization, and repressurization combined in various sequential arrangements and not necessarily in that order.

For instance, in U.S. Patent 3,176,444 a pressure swing adsorption process is disclosed wherein a feed gas is separated into a product and impurities which are adsorbed on the adsorbent in the various beds of the process. It is recited that adsorption is continued only so long as the adsorption front is still within the bed after which depressurization is performed cocurrently to utilize the rest of the adsorbent bed before desorption to remove impurities from the bed for the purpose of regeneration.

In another adsorptive scheme, U.S. Patent 3,226,913 discloses a separation process using pressure swings wherein the adsorption is terminated short of breakthrough of the bed and three stages of sequential depressurization are utilized in conjunction with a purge gas which constitutes preferably a separate purge medium.

U.S. Patent 3,430,418 provides an adsorptive separation wherein adsorption is conducted short of full use of the adsorption bed, cocurrent depressurization is performed wherein depressurized gas is used immediately to repressurize a second bed, and finally additional steps of depressurization are performed to regenerate the bed.

U.S. Patent 3,751,878 discloses a process for the separation of methane from carbon dioxide in a feed gas mixture using an adsorptive separation technique including an adsorption step, a carbon dioxide rinse step and a pressure reduction step.

In U.S. Patent 3,977,845 an adsorptive separation technique is disclosed using an additional segregated adsorptive bed to recover a product gas and a low purity gas from a feed gas mixture.

U.S. Patent 4,013,429 describes a low pressure adsorptive separation of oxygen and nitrogen from air using the steps of adsorption, rinse, depressurization and repressurization.

U.S. Patent 4,000,990 discloses an adsorptive separation of landfill gas using three or more adsorptive beds and a pretreatment zone. The process uses double bed adsorption zones and does not include a rinse step between the adsorption and venting steps.

U.S. Patent 4,021,210 provides an adsorptive process using an adsorptive step, two depressurization steps and a purge step to remove residual components from the adsorptive media.

A separation of hydrogen and carbon dioxide or methane and carbon dioxide is disclosed in U.S. Patent 4,077,779 to a common assignee wherein an adsorptive separation is carried out with a series of parallel adsorptive beds of 4, 5 and 6 beds in which the following steps are performed: an adsorption step, a rinse step with secondary component, a depressurization step, an inert or air purge step, an evacuation step and a repressurization step. In order to practice the separation technique of this patent, it is necessary to perform two purges, one involving secondary product which is recovered from the gas mixture being separated and another purge that involves the use of an inert gas or an air purge which requires an outside gas source to be utilized and results in at least a temporary contamination of the regenerating bed being purged.

U.S. 4,171,206 discloses a process for resolving a mixture of hydrogen and carbon dioxide and a tertiary product of carbon monoxide and methane. The process uses the steps of adsorption, rinse, desorption, evacuation, pressure equalization, purging and repressurization.

U.S. 4,171,207 describes a process for the adsorptive separation of hydrogen from methane and a tertiary product containing ethane and higher hydrocarbons. The process includes the steps of adsorption, rinse, pressure equalization, depressurization, rinse, evacuation and repressurization.

U.S. 4,264,340 is an improvement over previously cited U.S. 4,013,429 wherein air at low pressure is separated into nitrogen and oxygen and the nitrogen is dried in a thermal swing unit after recovery to provide a dry nitrogen product.

In contrast to the prior art, the present invention provides a viable secondary product as well as an unexpected enhancement of the secondary product recovery (carbon dioxide) which is particularly amenable to processes such as certain processes for separation of the gases produced during enhanced recovery of petroleum, petrochemical processes, natural gas processing or landfill gas applications, wherein the carbon dioxide is a potential product or could be utilized for petroleum reservoir pressure enhancement. The present invention also constitutes an improvement over the prior art in capital costs of an installation, while offering process simplicity by the reduction in the number of steps and the amount of gaseous materials necessary to operate the process. The problems attendant with the prior art, particularly of low recovery of secondary component of a gas mixture in an adsorptive separation, are overcome by the present invention, which will be described in greater detail below.

## BRIEF SUMMARY OF THE INVENTION

The present invention is directed to a process for the adsorptive separation at high pressure of a gas mixture selected from the primary/secondary component group consisting of hydrogen and carbon dioxide, methane and carbon dioxide, nitrogen and methane, nitrogen and carbon dioxide or hydrogen and nitrogen using an adsorbent having a selectivity greater than 20 for the secondary component over the entire range of pressure and temperature in which the adsorptive separation is performed and each component is recovered at high recovery, high purity and the primary component is recovered at elevated pressure in a plurality of parallel adsorption beds which are preferentially selective to the adsorption of the secondary component comprising the steps of, passing a feed gas mixture at a pressure of 30-500 psig into one of the plurality of adsorption beds wherein primary component is produced from the bed as a pure product and secondary component is selectively adsorbed on the adsorbent wherein the adsorbent has a selectivity of greater than 20 for the secondary component separated and at the gas composition, pressure and temperature of operation of the adsorbent; discontinuing adsorption and rinsing the adsorption bed with a stream of secondary component at approximately the feed pressure to remove any primary component as an effluent which is recycled to the feed to the process; depressurizing the rinse adsorption bed to an intermediate pressure countercurrent to said adsorption to at least partially remove the secondary component from said adsorption bed; following said depressurization and without further rinsing, evacuating said adsorption bed countercurrently to a subatmospheric pressure to further remove secondary component from said bed, and repressurizing the bed to superatmospheric pressure with primary component to prepare the bed for another adsorption step.

Preferably, the process is practiced wherein the gas mixture to be separated comprises methane as a primary component and carbon dioxide as a secondary component. Such a gas mixture is typically recovered from landfill gas and produced gas from oil fields that are being oxygen fireflooded or carbon dioxide flooded. Alternately, the primary component can be hydrogen or nitrogen and the secondary component can be carbon dioxide methane when the primary component is nitrogen or nitrogen when the primary component is hydrogen.

The process can be practiced using at least one adsorbent bed preferably in a 4, 5 or 6 parallel bed configuration.

The process provides for recovery of both primary and secondary component at 95% or above. Preferably, the process is operated to recover 98% or better of both the primary and secondary component with a purity of 98% or better for both the primary and secondary component.

Preferably, the feed gas mixture is at a pressure in the range of 30-500 psig, optimally approximately 80-150 psia and at a temperature in the range of 10°C to 60°C, optimally approximately 20°C.

The feed gas mixture is preferably pretreated to remove water, heavy hydrocarbons and other trace impurities prior to separation of the primary and secondary components in the adsorptive beds.

Optimally, the rinse of the adsorptive bed with secondary component is performed cocurrently to the adsorption step of the process at approximately adsorption pressure. Additionally, the repressurization of the bed is performed with primary component countercurrently to the adsorption of the same bed.

## BRIEF DESCRIPTION OF THE DRAWINGS

FIG 1 is a schematic flow diagram of a 5 bed embodiment of the present invention.

FIG 2 is a graph of selectivity of nitrogen from air using sodium mordenite adsorbent at several pressures.

FIG 3 is a graph of selectivity of carbon dioxide from a 50-50 mixture of carbon dioxide-methane using BPL carbon adsorbent at three pressures.

FIG 4 is a schematic longitudinal section of an adsorbent bed showing the profiles of different variables as a function of bed length at a given time.

## DETAILED DESCRIPTION OF THE INVENTION

Upgrading of low BTU gas streams is becoming increasingly important with the advent of various petroleum production pressure-assisted recovery schemes. Such schemes include the recovery of natural gas or methane from landfill sites, as well as from the gases obtained during the enhanced recovery of non-naturally producing petroleum fields, such as $CO_2$ flood operations and oxygen fireflood. Additionally, some industrially pro duced hydrogen streams such as steam reformer off-gas also contain carbon dioxide as a bulk impurity which requires upgrading for commercial application. Typically, such low BTU streams can include approximately 50% methane and 50% carbon dioxide, although the contents can vary considerably and other impurities will be present depending upon the site conditions and the particular source of the low BTU fuel gas resource. Methane and carbon dioxide mixtures offer an attractive commodity for upgrading, but require the performance of a difficult separation. In known prior art cryogenic techniques, separation of methane and carbon dioxide is extremely difficult due to the close relative volatility. Such prior art cryogenic techniques have typically utilized additional additives or solvent extractants in order to enhance the separation of the two components under cryogenic conditions. The steam-methane reformer off-gases can typically contain up to 30% carbon dioxide in hydrogen. In known techniques, hydrogen recoveries have generally been only in the 65 to 85% range.

The present invention uses a non-cryogenic adsorptive separation wherein a marketable high purity methane, hydrogen or nitrogen primary component is recovered in good quantity and a high purity carbon dioxide, methane or nitrogen by-product or secondary component is recovered in good quantity with potential for use or venting depending upon the economic conditions at the site of the gas processing. The preferred gas mixtures susceptible of separation with the present invention are $CH_4/CO_2$, $H_2/CO_2$, $N_2/CH_4$ and $H_2/N_2$ where the primary component is listed first and the secondary component is listed second. The key to the high recovery of the methane, hydrogen or nitrogen product and the high purity of the carbon dioxide, methane or nitrogen of the secondary product is the high pressure secondary component rinse step of the adsorptive process.

The invention utilizes packed beds of molecular sieve zeolite or activated carbon material which is selective to the adsorption of secondary component, such as carbon dioxide, methane or nitrogen in relation to the primary component, such as methane, hydrogen or nitrogen of the feed gas mixture. In this manner, the primary component is allowed to pass through the various adsorptive beds, while the secondary component is adsorbed onto the adsorbent of the various beds.

The adsorbents which may be utilized in the present invention include various molecular sieve A, X and Y zeolites, mordenites and clinoptilolites with sodium, calcium or other cations or zeolites which have been exchanged with two different cations, as well as various activated carbons. However, these adsorbents must be carefully selected for the gas mixtures being processed so that at high pressure operation, such as is desired in the mode of operation of the present invention, the selectivity of the adsorbent for the adsorbed gas species of the gas mixtures is always greater than 20. Adsorbents with high selectivity are necessary, particularly with high pressure separation operation because the adsorbent selectivities effect three important characteristics of an efficient separation process such as (a) increased production capacity which reduces adsorbent inventory, (b) low ratio of high pressure rinse/product gas quantity which reduces the energy of separation due to less compression power requirement for the rinse gas, and (c) increased recovery of the components of the gas mixture being resolved. Selectivity(S) is defined by the following

formula $S = (n_1 Y_2 / n_2 Y_1)$, wherein $n_1$ and $n_2$ are the specific amounts of component 1 and component 2 respectively adsorbed and $Y_1$ and $Y_2$ are the equilibrium gas mole fractions of each respective component. S is usually a function of pressure (P), temperature (T), and gas composition ($Y_1$). S usually decreases with increasing P, T and $Y_1$. Operation at higher pressure (lower S) increases the coadsorption of the primary gas component, which is not desired to be adsorbed in the adsorber, which in turn increases the quantity of high pressure purge gas (rinse) necessary for the displacement of co-adsorbed and void-space primary component by the secondary component.

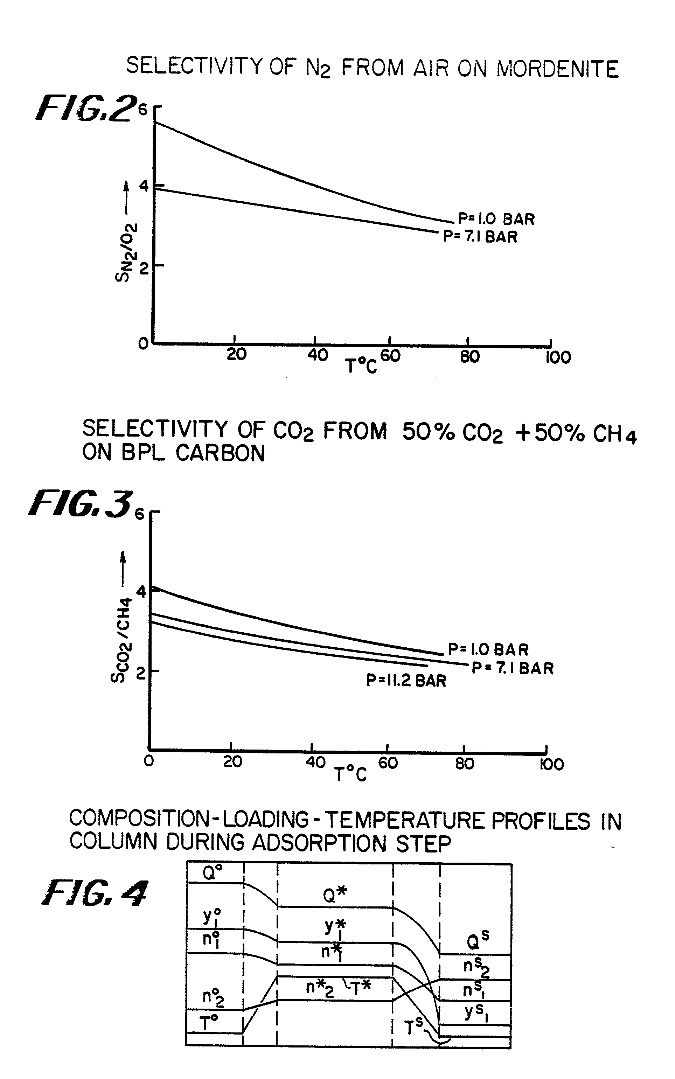

For example, FIG 2 and 3 respectively show S for a nitrogen/oxygen gas mixture adsorbed on sodium mordenite and a carbon dioxide/methane gas mixture adsorbed on BPL carbon as functions of P and T for a given $Y_1$. As shown in these figures, S decreases with increasing P and T. In FIG 2 at 20°C, S decreases from 4.8 to 3.5 as the pressure increases from 1.0 to 7.1 bar. In FIG 3 at 20°C, S also decreases from 3.5 to 3.0 as the pressure increases from 1.0 to 7.1 bar. These reductions in selectivity(S) with increasing pressure require increased high pressure purge gas quantities (rinse) of the secondary component. Accordingly, it is necessary for the efficient operation of the high pressure adsorptive separation of the present invention to utilize only adsorbents having a high selectivity greater than 20 for the secondary gas components in the appropriate gas feed mixture to be separated at the pressure and temperature ranges dictated by conditions within the adsorption bed. Therefore, at high pressure operation, high selectivity is critical to the economic and efficient operation of the adsorptive separation.

Various adsorbents may be utilized in the process of the present invention when their selectivity, as defined above for the given separation system, is taken into consideration, and only those adsorbents for that system are utilized having selectivity greater than 20. Appropriate adsorbents include various cation exchanged A, X and Y zeolites, mordenites and various activated carbons. Table 1-A below shows various selectivity numbers, which are dimensionless, for an adsorption process selective to carbon dioxide from a gas mixture of 50% carbon dioxide and 50% methane. For a given temperature range and pressure range it is shown that calcium 5A zeolite, sodium X zeolite, sodium Y zeolite and sodium mordenite all provide high selectivities for the intended separation and are therefore relevant to the present invention.

## T A B L E    1-A

Selectivities for Adsorption of $CO_2$ from a 50% $CO_2$ + 50% $CH_4$
Mixture on Various Adsorbents Under Different Conditions

| Temperature (°K) | Pressure (Bar) | | |
|---|---|---|---|
| | 5 | 10 | 15 |
| **Ca 5A Zeolite** | | | |
| 300 | 327 | 308 | 297 |
| 311 | 287 | 272 | 263 |
| 322 | 252 | 242 | 233 |
| 339 | 206 | 198 | 193 |
| **NaX Zeolite** | | | |
| 300 | 250 | 228 | – |
| 311 | 180 | 162 | – |
| 322 | 135 | 120 | – |
| 339 | 93 | 81. | – |
| **NaY Zeolite** | | | |
| 300 | 181 | 182 | 183 |
| 311 | 136 | 136 | 136 |
| 322 | 105 | 104 | 104 |
| 339 | 73 | 72 | 71 |
| **Na Mordenite** | | | |
| 300 | 243 | 223 | 213 |
| 311 | 223 | 205 | 195 |
| 322 | 207 | 189 | 180 |
| 339 | 187 | 170 | 161 |

Table 1-B shows a similar array of the selectivities of adsorption of carbon dioxide from nitrogen under various conditions of pressure and temperature. The table shows only two examples of adsorbents. Other adsorbents such as X and Y zeolite can be used for this system but their selectivities are extremely large, generally greater than 2000 under similar conditions.

T A B L E     1-B

Selectivities of Adsorption for $CO_2$ from a 50% $CO_2$ + 50% $N_2$
Mixture on Various Adsorbents Under Different Conditions

| Temperature | Pressure (Bar) | | |
|---|---|---|---|
| (°K) | 5 | 10 | 15 |
| **5A Zeolite** | | | |
| 300 | 1325 | 1350 | 1350 |
| 311 | 1090 | 1090 | 1090 |
| 322 | 915 | 915 | 915 |
| 339 | 710 | 710 | 710 |
| **Na Mordenite** | | | |
| 300 | 532 | 486 | 461 |
| 311 | 496 | 452 | 428 |
| 322 | 464 | 422 | 400 |
| 339 | 423 | 384 | 364 |

Table 1-C shows yet another array of selectivities of adsorption for, in this case, nitrogen from hydrogen under various conditions of pressure and temperature.

T A B L E     1-C

Selectivities of Adsorption for $N_2$ from a 50% $N_2$ + 50% $H_2$
Mixture on Various Adsorbents Under Different Conditions

| Temperature | Pressure (Bar) | | |
|---|---|---|---|
| (°K) | 5 | 10 | 15 |
| **CaX Zeolite** | | | |
| 300 | 83 | 76 | 73 |
| 311 | 74 | 67 | 64 |
| 322 | 66 | 60 | 56 |
| 339 | 57 | 51 | 48 |
| **Na Mordenite** | | | |
| 300 | 33 | 26 | 22 |
| 311 | 28 | 23 | 21 |
| 322 | 23 | 20 | 20 |

Because temperature is one of several factors which effects the selectivity of given adsorptive separation, it is also noted that column temperature for separation of a bulk binary mixture rises due to generation of the heat of adsorption. As a result, the loading profiles ($n_1$ and $n_2$) in a column as a function of distance (x) from the bed inlet, the horizontal distance from one solid vertical line to the other solid vertical line of Fig 4 take the shapes shown in FIG 4 during the adsorption step. FIG 4 diagramatically shows the profiles of various parameters in a column wherein the feed end of the column is represented by the left hand side of the Figure and the downstream or effluent end of the column is represented by the right hand side of the Figure. The variable x represents the distance from the inlet end of the packed bed adsorption col umn, although not labeled as such in FIG 4. The Q line represents gas flow rate, while the $Y_1$ line represents the gas composition. The $n_1$ represents the loading of the one component (secondary) while $n_2$ represents the loading of the other component (primary). T represents the temperature in the adsorption bed. A large section of the column (80-95%) attains a temperature $T^*$ which is much higher than the feed gas or initial column temperature ($T^\circ$ or $T^s$). The gas composition in that section is $Y_1^*$. Consequently, the specific amounts adsorbed ($n_1^*$ and $n_2^*$) of the components of the mixture to be separated in a major portion of the column are in equilibrium at $P$, $T^*$ and $Y_1^*$ and not at $P$, $T^\circ$ and $Y_1^\circ$ which are the feed gas conditions. Thus, $n_1^*$ is much lower than $n_1^\circ$, the specific amounts adsorbed at feed conditions. This can cause the selectivity of separation ($S^* = n_1^* Y_2^* / Y_1^* n_2^*$) at $P$, $T^*$ and $Y_1^*$ to be much less than that at the feed conditions. Consequently, a larger amount of $n_2^*$ or the quantity of the primary component is coadsorbed compared to the amount under isothermal operation. This coadsorbed primary component ($n_2^*$) and that in the void gas is purged out during the secondary component rinse step of the process. Therefore, higher $n_2^*$ (or lower $S^*$) and higher $P$ (lower $S$ and more void gas in column) increase high pressure purge gas requirements (rinse) of the process. It is, therefore, desirable that adsorbents which have high selectivity ($S$ greater than 20) in the entire $P$ and $T$ ranges of the operation of the column be used for separation of gas mixtures using the process so that $n_2^*$ (primary component co-adsorption) is low and purge gas quantity is small.

Tables 2, 3, 4 and 5 show the effect of various adsorbents on various gas mixture separations at low and high pressures. Table 2 indicates that separation of air using mordenite is detrimentally effected by high pressure operation, as shown in the decrease in nitrogen product and the dramatic increase in the ratio of nitrogen rinse to nitrogen product required to operate the system. Table 3 identifies that the separation of carbon dioxide from methane using activated carbon is also detrimentally effected by increase in the pressure of operation, whereby it is shown that the carbon dioxide product decreases and the ratio of carbon dioxide rinse to carbon dioxide product increases detrimentally. In comparison, Table 4 and Table 5 show the separation of carbon dioxide from methane using sodium X and sodium Y zeolites at different pressures, whereby higher pressure operation does not significantly effect carbon dioxide product or the carbon dioxide rinse to product ratio. The contrast produced by these tables is demonstrative of the effect resulted by practice of the present invention using adsorbents of high selectivity for a given gas mixture, pressure and temperature range.

The materials of Table 4 and 5 perform better at higher pressure because of the high selectivity of adsorption of carbon dioxide from methane over a large range of $P$ and $T$ which was earlier demonstrated in Table 1. Specifically identified adsorbents for this separation include the calcium A, sodium X, and sodium Y zeolites and sodium mordenite. Other ion exchange forms of A, X and Y zeolites and mordenite, such as single and binary ion exchange zeolites using ions from groups I and II of the metals of Periodic Table of the Elements may be utilized as long as the system and the pressure and temperature in which the adsorbent will be used results in the selection of an adsorbent having high selectivity for the separation to be performed, at least a selectivity greater than 20.

## Table 2: Separation of Air Using Mordenite

Feed Gas: 21% $O_2$ + 79% $N_2$
Temperature = 20°C

| Pressures (Bar) | | | | | | |
| Feed | Evacuation | $N_2$ Product | $\frac{N_2 \text{ Rinse}}{N_2 \text{ Product}}$ | $N_2$ Purity | $O_2$ Purity | $N_2$ Recovery |
|---|---|---|---|---|---|---|
| 1.0 | 0.1 | 0.26* | 0.85 | 99% | 90% | 88% |
| 7.1 | 0.7 | 0.22 | 3.77 | 99% | 90% | 42% |

## Table 3:   Separation of $CO_2/CH_4$ Using BPL Carbon

Feed Gas:   50% $CO_2$ + 50% $CH_4$
Temperature = 20°C

| Pressures (Bar) | | $CO_2$ Product | $\dfrac{CO_2\ Rinse/}{CO_2\ Product}$ | $CO_2$ Purity | $CH_4$ Purity | $CO_2/CH_4$ Recovery |
|---|---|---|---|---|---|---|
| Feed | Evacuation | | | | | |
| 7.1 | 0.1 | 0.72* | 3.0 | 99% | 98.5% | 99% |
| 11.2 | 0.15 | 0.68 | 4.5 | 99% | 98.5% | 99% |

## Table 4:   Separation of $CO_2/CH_4$ Using NaX Zeolite

Feed Gas:   50% $CO_2$ + 50% $CH_4$
Temperature = 20°C

| Pressures (Bar) | | $CO_2$ Product | $\dfrac{CO_2\ Rinse/}{CO_2\ Product}$ | $CO_2$ Purity | $CH_4$ Purity | $CO_2/CH_4$ Recovery |
|---|---|---|---|---|---|---|
| Feed | Evacuation | | | | | |
| 7.1 | 0.1 | 0.85* | 0.66 | 99% | 98.5% | 99% |
| 11.2 | 0.15 | 1.06 | 0.56 | 99% | 98.5% | 99% |

## Table 5:   Separation of $CO_2/CH_4$ Using NaY Zeolite

Feed Gas:   50% $CO_2$ + 50% $CH_4$
Temperature = 20°C

| Pressures (Bar) | | $CO_2$ Product | $\dfrac{CO_2\ Rinse/}{CO_2\ Product}$ | $CO_2$ Purity | $CH_4$ Purity | $CO_2/CH_4$ Recovery |
|---|---|---|---|---|---|---|
| Feed | Evacuation | | | | | |
| 7.1 | 0.1 | 1.38* | 0.42 | 99% | 98.5% | 99% |
| 11.2 | 0.15 | 1.29 | 0.49 | 99% | 98.5% | 99% |

*All quantities are lb mole/1000 lbs of adsorbent/cycle

One example of the application of this process is separation of landfill gas effluent containing about 40-60% carbon dioxide, 60-40% methane, a large number of trace hydrocarbon impurities and some nitrogen and oxygen. The gas is also saturated with water. The feed gas after removal of the trace impurities and water is essentially a binary gas mixture of carbon dioxide and methane containing dilute amounts of nitrogen and oxygen. The mixture can be separated to produce a stream of approximately 98% + methane and a stream of approximately 98% + carbon dioxide with 98% + recovery of both components using the process described. It is, however, essential that the pressure swing adsorption process be carried out at elevated pressure (30-500 psig) because of the adsorption characteristics of carbon dioxide and methane and because the methane product is needed at high pressure and the separations of water and the trace impurities are facilitated at high pressure. Another example of an amenable gas mixture for separation is the separation of carbon dioxide/methane or nitrogen/methane mixtures at high pressure from an enhanced oil recovery operation where carbon dioxide or nitrogen is injected into the well to displace crude oil and/or associated natural gas. Separated carbon dioxide or nitrogen are recovered for reinjection. A third example is separation and recovery of carbon dioxide from a steam/methane reformer off-gas containing carbon dioxide, carbon monoxide, methane, hydrogen and nitrogen. All of these applications require operation of the pressure swing adsorptive separation scheme at elevated pressure. However, it is not obvious that operation of a pressure swing adsorption scheme at an elevated adsorption pressure will provide efficient separation of the gas mixture. It is especially not obvious that the process of this invention, which utilize a high pressure rinse step following the adsorption step will provide efficient separation. As demonstrated in the tables preceding, some gas mixtures using adsorbents in general are adversely affected by increased pressure operation when the process of this invention is employed. Therefore, low pressure operations are not exemplary of the operation of the present invention at high pressure for a given gas composition at pressure and temperature ranges dictated at the separation site.

The present invention will now be described with reference to a preferred embodiment.

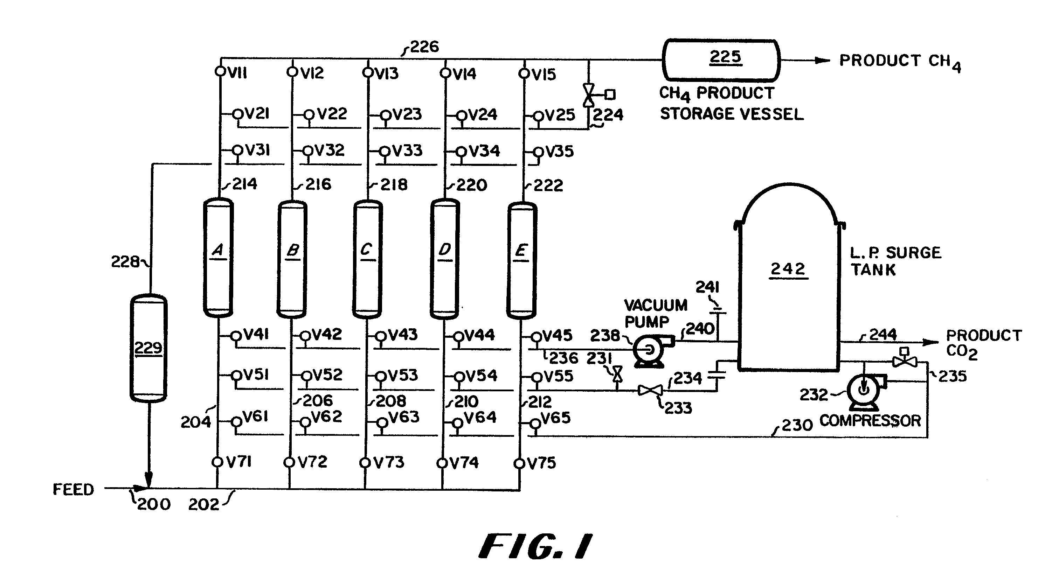

Referring to FIG 1, a 5 bed configuration of the process of the present invention is illustrated. A raw landfill gas is collected and compressed to 30-500 psig via a feed compressor after which the gas stream is cooled to near ambient temperature in a cooler-condenser and any condensate is removed as a liquid in a knock-out drum. The raw gas is then treated for removal of trace chemical impurities and dried to a dew point of approximately +10° to -70°F in a pretreatment system. None of this apparatus is shown in the diagrams, but such pretreatment is typical for such adsorptive systems as well documented in the prior art. Pretreated feed as described above is introduced in line 200 at a pressure in the range of approximately 30-500 psig, optimally 70-150 psig. It is combined with a recycle gas from a rinsing bed in line 228 and vessel 229. The combined gas in manifold 202 is introduced into any one of the 5 beds, A, B, C, D and E. For purposes of this discussion, the description of the sequences in bed A will be set forth. In this case, adsorption is performed by passing the gas through line 204 into bed A and evolving a high purity methane effluent in line 214. It is understood that beds B, C, D and E operate through lines 206, 208, 210, 212, 216, 218, 220 and 222 in a similar manner. The feed gas passes through open valve 71 and produces a methane product through open valve 11. This gas is then collected and removed as product in line 226 to the methane product storage vessel 225. The storage vessel can be optional. Methane can be withdrawn as a primary component product. A portion of the gas in line 226 or vessel 225 is used for the repressurization step. When the carbon dioxide, which is selectively adsorbed on an adsorbent component in bed A reaches its saturation with the wave front near the downstream end of bed A, the adsorption step is terminated.

Bed A is then cocurrently purged with a high pressure carbon dioxide rinse gas at the feed pressure in the range of 30-500 psig. High purity carbon dioxide is removed from the surge tank 242 and compressed by the carbon dioxide rinse compressor 232 to an elevated pressure of approximately the same pressure as the feed gas. The gas is passed through line 230 and open valve 61 wherein the carbon dioxide rinse passes con currently through bed A to remove any remaining methane in the form of void gas or co-adsorbed gas on the adsorbent. The rinse step is continued until most of the residual methane has been removed from bed A and the carbon dioxide is approximately at breakthrough at the downstream end of bed A. The removed void gas and co-adsorbed methane gas containing a mixture of methane and carbon dioxide of similar composition to the feed gas pass through open valve 31 and are recycled in line 228 and vessel 229 to be blended with the feed in line 200. This gas varies in purity as it comes off of bed A and the mixing vessel 229 can be used to blend the recycle to the feed line 200. When the carbon dioxide is approximately at the breakthrough point in bed A, the rinse step is terminated. Bed A is now saturated with high pressure carbon dioxide and is ready for the desorption and depressurization step. With the appropriate change in valves, the carbon dioxide is desorbed by depressurization countercurrently in bed A through open valve 51. The gas is removed in line 234 through open valve 233 and is collected in the surge tank 242. The use of the surge tank may be optional. When the desorptive depressurization reaches the desired intermediate (atmospheric) pressure, Bed A is then switched to the evacuation sequence.

No inert gas or air purge is performed between depressurization and evacuation as in the prior art. This achieves a higher recovery of the secondary component or carbon dioxide gas and improves the stability and flexibility of the overall operation. The evacuation is initiated immediately after the desorptive depressurization step and is achieved by evacuating bed A countercurrently through open valve 41 in line 236, wherein the vacuum system 238 delivers the evacuated low pressure carbon dioxide through line 240 to the surge tank 242. The evacuated and depressurized carbon dioxide gas phases which are combined in the surge tank constitute the secondary product or by-product which can be used for rinse with the net production recovered through line 244. Again, the evacuation is conducted until a desired subatmospheric pressure is reached, preferably in the range of 1-2.5 psia or approximately 50-130 torr. The depressurization effluent can be directly compressed as it evolves and used as the high pressure rinse gas without the use of the surge tank or using a smaller surge tank.

It is also possible to operate the five bed system by collecting only the evacuated carbon dioxide and venting through vent 231 that gas which is produced during the desorption step. This will result in a smaller amount of a higher purity carbon dioxide by-product which in turn will improve the methane recovery. Alternately, some venting can occur through vent 241.

After the low pressure evacuation is terminated, repressurization is performed in bed A to bring it back to feed condition. Repressurization is done countercurrently by admitting some of the primary product from one of the other beds through open valve 21 and line 224 to deliver high pressure methane at feed pressure conditions into bed A until the pressure of the bed approximates feed conditions in the range of 30-500 psig. This drives any residual carbon dioxide to the front end of the bed and places the bed in condition for a renewed adsorption sequence. Each of the other beds, B, C, D and E goes through a similar cycle and in operating in conjunction with one another provide a continuous product flow of methane and a by-product flow of carbon dioxide. Cycle times for the 5 bed configuration are identified at the top of Table 6 below. The cycle time of 15 minutes shown in Table 6 is arbitrary and other cycle times can be chosen. Table 6 additionally shows the valve position for the various valves of FIG 1 throughout the cycle sequence. At the bottom of Table 6 a typical representation of the sequence for bed A is set forth. Again, this process is capable of producing high purity methane at high recovery, as well as high purity carbon dioxide at high recovery, wherein the purity of the carbon dioxide is 98% + and the recovery of the carbon dioxide is 98% +. The prior art, such as in U.S. Patent 4,077,779 is unable to achieve such a high recovery of carbon dioxide.

## TABLE 6

### Valve Positions PSA-5

| Valve | Time (Min.) | | | | |
|---|---|---|---|---|---|
| | 1-3 | 3-6 | 6-9 | 9-12 | 12-15 |
| 11 | 0 | X | X | X | X |
| 12 | X | X | X | X | 0 |
| 13 | X | X | X | 0 | X |
| 14 | X | X | 0 | X | X |
| 15 | X | 0 | X | X | X |
| 21 | X | X | X | X | 0 |
| 22 | X | X | X | 0 | X |
| 23 | X | X | 0 | X | X |
| 24 | X | 0 | X | X | X |
| 25 | 0 | X | X | X | X |
| 31 | X | 0 | X | X | X |
| 32 | 0 | X | X | X | X |
| 33 | X | X | X | X | 0 |
| 34 | X | X | X | 0 | X |
| 35 | X | X | 0 | X | X |
| 41 | X | X | X | 0 | X |
| 42 | X | X | 0 | X | X |
| 43 | X | 0 | X | X | X |
| 44 | 0 | X | X | X | X |
| 45 | X | X | X | X | 0 |
| 51 | X | X | 0 | X | X |
| 52 | X | 0 | X | X | X |
| 53 | 0 | X | X | X | X |
| 54 | X | X | X | X | 0 |
| 55 | X | X | X | 0 | X |
| 61 | X | 0 | X | X | X |
| 62 | 0 | X | X | X | X |
| 63 | X | X | X | X | 0 |
| 64 | X | X | X | 0 | X |
| 65 | X | X | 0 | X | X |
| 71 | 0 | X | X | X | X |
| 72 | X | X | X | X | 0 |
| 73 | X | X | X | 0 | X |
| 74 | X | X | 0 | X | X |
| 75 | X | 0 | X | X | X |
| Bed A → | Adsorption | H.P. Rinse | Depress | Evac | Repress |

The present invention realizes improved recovery of secondary product or carbon dioxide at an unexpected magnitude. This is achieved with a lower overall capital cost for an installation, which is capable of operating under the present invention's process sequence. Although the omission of the inert purge or air purge required of the prior art might seem to lead one to a higher recovery of secondary product, there are systems which would not allow for such an omission, such as gas separations of methane and normal butane where the secondary component is relatively more strongly adsorbed than carbon dioxide. Further the magnitude of improvement in secondary product recovery defined as moles of component in final product divided by moles of component in fresh feed, could never have been expected. Selectivity also plays a major role in obtaining high recoveries at high efficiency and high pressure. Choosing adsorbents with selectivity above 20 for the given system and required conditions is essential to the present invention operating at relatively high pressures.

With reference to Table 7 below, it can be seen that a comparison of the present invention involving 5 steps in a cycle sequence per bed in contrast to the prior art U.S. Patent 4,077,779 using 6 steps in a cycle sequence per bed, produces the equivalent methane purity and recovery of 99%, but an overwhelming improvement is seen in the carbon dioxide recovery, although both the present invention and the prior art maintain a carbon dioxide purity of 99%. The insensitivity of these parameters of methane recovery and purity and carbon dioxide purity along with the heightened achievement in the parameter of carbon dioxide recovery is surprising and is not general to all chemical systems as noted above for the methane/normal butane systems.

A drawback to the method of U.S. Patent 4,077,779, as applied to the separation of methane and carbon dioxide, exists in the amount of carbon dioxide recovered during depressurization versus the amount necessary for high pressure rinse. For operational stability and peak recovery, it is necessary to remove more carbon dioxide during depressurization than is utilized in carbon dioxide high pressure rinse of another bed. As set forth in Table 7, the present invention enjoys a dramatic magnitude of recovery of carbon dioxide while the prior art in U.S. Patent 4,077,779 has a marginal net recovery of 5%.

## TABLE 7

### 2MMSCFD Landfill Gas PSA
### Operating Pressure = 70 psig

|  | | CH$_4$ Recovery | CH$_4$ Purity | CO$_2$ Recovery | CO$_2$ Purity | Relative Estimated[a] Power | Relative System Capital |
|---|---|---|---|---|---|---|---|
| 1. | Basic Process (A,C,E,F) | 80% | 99% | 99% | 79% | 1.0 | 1.0 |
| 2. | Present Invention (A,B,C,E,F) | 99% | 99% | 99% | 99% | 1.59 | 1.35 |
| 3. | U.S. Pat. 4,077,779 (A,B,C,D,E,F) | 99% | 99% | 5% | 99% | 1.37 | 1.38 |

KEY: Process Steps

A – Adsorption
B – High Pressure CO$_2$ Rinse
C – Desorption
D – Air/Inert Gas Purge
E – Evacuation
F – Repressurization

[a] Power numbers do not include feed or product compression

Therefore, in designing a commercial installation for a methane/carbon dioxide separation, such as a system for the recovery of methane from landfill gas, the comfortable margin of carbon dioxide recovery provided by the present invention provides a dramatic distinction between it and the process of the prior art in U.S. Patent 4,077,779. At some feed pressures, when 99% methane purity and recovery is required, the 6 step sequence of the prior art requires more secondary component for the high pressure rinse step than is made available through the secondary component depressurization recovery. Such a short fall would begin to effect methane recovery over a series of cycle sequences. Such a shortcoming is not exhibited by the present invention, as set forth herein. This dramatic increase in operational capability of the 5 step adsorptive process of the present invention in contrast to the 6 step adsorptive process of U.S. Patent 4,077,779 indicates that the deletion of an inert or air purge in such an adsorptive process leads to an unexpected and significant improvement in the art of adsorptive separation of methane and carbon dioxide gas mixtures when conducted in cooperation with the selection of an adsorbent having high selectivity, i.e. S > 20 for the system contemplated.

An advantage of the present invention over another prior art process set forth in U.S. Patent 4,013,429 is high pressure operation with maintained recovery and efficiencies. The '429 patent includes steps of adsorption, rinse, evacuation and repressurization. However, when the separation of the '429 patent is applied to high pressure conditions as are required by the present invention, the absence of a teaching to use high selectivity adsorbents for given gas separations results in the effects shown in previously discussed Table 2. In other words, product quality decreases and rinse to product ratios go up dramatically as high pressure operation is implemented. High rinse/product ratios have very adverse economic consequences - high power consumption and increased equipment sizes. Using the parameters of the present invention wherein the adsorbent system is limited to high selectivity adsorbents for the given gas system being separated, high pressure operation will be possible, whereby the result of high pressure separated product is obtained without a loss in the quality or quantity of secondary product also produced.

The process advantages of the present invention have been documented in laboratory experiments designed to model the adsorptive separation sequence of the proposed commercial scale process of the present invention. Such a demonstrative example is set forth below.

## EXAMPLE

A bench scale unit was run to evaluate the performance of the above mentioned PSA process. A feed gas comprising 42.5% $CO_2$ and 57.5% $CH_4$ at 70 psig pressure and 21°F temperature was fed to a column containing 7 lbs. of NaX zeolite as the adsorbent. A $CH_4$ enriched stream containing 99% $CH_4$ was withdrawn from the column at 69 psig. 0.013 lb. moles of feed gas was passed through the column during the adsorption step. The $CO_2$ rinse step was carried out using a 99% $CO_2$ gas at 72 psig and the effluent was recycled as feed for the adsorption step. The column was then depressurized to ambient pressure level and then evacuated to 95 torr. The effluent was 98.5% $CO_2$, part of which was withdrawn as $CO_2$ product and the other part was recompressed and recycled as $CO_2$ rinse. The column was repressurized to 70 psig after evacuation using part of the 99% $CH_4$ product and the cycle was repeated. The amounts of the $CH_4$ and $CO_2$ products form the system were respectively 0.0074 and 0.0056 lb. moles. The purities of both products were 99%. Thus, the product recovery for both constituents of the feed mixture were 99% (within experimental error).

In summary, the present invention discloses the use of a high selectivity adsorbent for efficient high pressure operation of a pressure swing adsorption gas separation process consisting of the steps of (a) adsorption at high pressure to produce a product stream rich in the less strongly adsorbed species of the feed gas mixture, primary component, (b) high pressure rinse with more selectively adsorbed component, secondary component, of the feed gas mixture with recycle of the effluent as feed, (c) depressurization to ambient pressure with all or part of the desorbed gas to be used as rinse gas in step (b), and the other part withdrawn as net product rich in more strongly adsorbed component, (d) evacuation to a subatmospheric pressure level and withdrawing the effluent as part of the more strongly adsorbed species product, and (e) pressurization to feed gas pressure using part of the effluent from step (a). The unique combination of the omission of a separate purge step and the requirement of an adsorbent for the gas mixture to be separated having a selectivity of greater than 20 provides unique and un expected results enjoyed by the present invention over the known prior art.

The present invention has been described above with several specific embodiments, but the scope of the present invention should be ascertained from the claims which follow.

## Claims

1. A process for the adsorptive separation at high pressure of a gas mixture selected from the primary/secondary component group consisting of hydrogen and carbon dioxide, methane and carbon dioxide, nitrogen and methane, nitrogen and carbon dioxide or hydrogen and nitrogen using an adsorbent having a selectivity greater than 20 for the secondary component over the entire range of pressure and temperature in which the adsorptive separation is performed and each component is recovered at high recovery, high purity and the primary component is recovered at elevated pressure in a plurality of parallel adsorption beds which are preferentially selective to the adsorption of the secondary component, comprising the steps of:

(a) passing a feed gas mixture at a pressure of 30-500 psig into one of the plurality of adsorption beds wherein primary component is produced from the bed as a pure product and secondary component is selectively adsorbed on the adsorbent wherein the adsorbent has a selectivity of greater than 20 for the secondary components separated and at the gas composition, pressure and temperature of operation of the adsorption bed;

(b) discontinuing adsorption and rinsing the adsorption bed with a stream of secondary component at approximately the feed pressure to remove any primary component as an effluent which is recycled to the feed to the process;

(c) depressurizing the rinsed adsorption bed to an intermediate pressure countercurrent to said adsorption to at least partially remove the secondary component from said adsorption bed;

(d) following said depressurization of step (c) and without further rinsing, evacuating said adsorption bed countercurrently to a subatmospheric pressure to further remove secondary component from said bed; and

(e) repressurizing the bed to superatmospheric pressure to prepare the bed for another adsorption step.

2. The process of Claim 1 wherein the temperature in the adsorptive bed during adsorption is in the range of 10°C to 60°C.

3. The process of Claim 1 wherein the separation is performed in adsorption beds numbering between two and six beds connected in parallel.

4. The process of Claim 1 wherein the recovery of primary and secondary component is 95% or above.

5. The process of Claim 1 wherein the feed gas mixture is landfill off-gas.

6. The process of Claim 1 wherein the feed gas mixture is steam reformer off-gas containing hydrogen as a primary component and carbon dioxide as a secondary component.

7. The process of Claim 1 wherein the feed gas mixture is produced gas from an oil field produced by an oxygen fireflood or carbon dioxide flood.

8. The process of Claim 1 wherein the feed gas mixture is pre-treated to remove water and heavy hydrocarbons.

9. The process of Claim 1 wherein the rinse of step (b) is performed cocurrently to the adsorption of step (a).

10. The process of Claim 1 wherein the repressurization of step (e) is performed countercurrently to the adsorption of step (a).

11. The process of Claim 1 wherein a portion of the secondary component evolved in steps (c) and (d) is used as the rinse gas of step (b).

12. The process of Claim 1 wherein a portion of the secondary component evolved in step (d) is used as the rinse gas in step (b).

13. The process of Claim 1 wherein step (c) is performed in at least two stages.

14. The process of Claim 1 wherein the intermediate pressure of step (c) is approximately atmospheric pressure.

15. The process of Claim 14 wherein the secondary component from the first stage of depressurization is used for the rinse of step (b).

16. The process of Claim 1 wherein when the gas mixture is methane and carbon dioxide and the adsorbent is selected from the group consistting of calcium A zeolite, sodium X zeolite, sodium Y zeolite or sodium mordenite.

FIG. I

0 257 493

SELECTIVITY OF $N_2$ FROM AIR ON MORDENITE

**FIG.2**

P=1.0 BAR

P=7.1 BAR

SELECTIVITY OF $CO_2$ FROM 50% $CO_2$ +50% $CH_4$ ON BPL CARBON

**FIG.3**

P=1.0 BAR

P=7.1 BAR

P=11.2 BAR

COMPOSITION-LOADING-TEMPERATURE PROFILES IN COLUMN DURING ADSORPTION STEP

**FIG.4**

## DOCUMENTS CONSIDERED TO BE RELEVANT

| Category | Citation of document with indication. where appropriate, of relevant passages | Relevant to claim | CLASSIFICATION OF THE APPLICATION (Int Cl 4) |
|---|---|---|---|
| X | JP-A-61 176 540 (AIR PRODUCTS) (08-08-1986) * Whole document * & EP-A-0 193 716 (10-09-1986) | 1-16 | B 01 D 53/04<br>C 01 B 3/56<br>C 01 B 21/04<br>C 01 B 31/20<br>C 07 C 9/04 |
| A | EP-A-0 164 024 (AIR PRODUCTS) * Page 17, lines 4-12 * | 1 | |

TECHNICAL FIELDS SEARCHED (Int. Cl.4)

B 01 D 53/00

The present search report has been drawn up for all claims

| Place of search | Date of completion of the search | Examiner |
|---|---|---|
| THE HAGUE | 13-11-1987 | KANOLDT W.W. |

EPO Form 1503 03 82

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document